# EUROPEAN PATENT APPLICATION

(11) **EP 3 067 687 A1**
(43) Date of publication of application: **14.09.2016**
(21) Application number: 14860495.2
(22) Date of filing: 05.03.2014
(51) Int. Cl.: G01N 21/33, G01N 21/85

(54) **EGG INSPECTION DEVICE AND METHOD**

(30) Priority: 09.11.2013 JP 2013232618
(71) Applicant: Nabel Co., Ltd., Kyoto-shi Kyoto 601-8444 (JP)
(72) Inventor: FUJITANI, Shinichi, Kyoto-shi Kyoto 601-8444 (JP)
(74) Representative: Gulde & Partner
(86) International application number: PCT/JP2014/055660
(87) International publication number: WO 2015/068408

(57) **Abstract**

An egg inspection apparatus to perform egg inspection based on the presence of adherence of protein to an eggshell is provided. According to the present invention, an egg inspection apparatus is provided that includes: a light radiating unit to radiate radiation light including an ultraviolet light component having a wavelength in an ultraviolet region to an eggshell surface of an egg subjected to inspection; a light receiving unit to receive reflection light reflecting from the eggshell surface out of the radiation light; and a determination unit to determine a state of adherence of protein to the eggshell surface based on the ultraviolet light component out of the reflection light received by the light receiving unit.

## Description

### Technical Field

The present invention relates to an egg inspection apparatus and method that are capable of inspecting a state of adherence of protein to an eggshell surface and the like.

### Background Art

Eggs represented by hen eggs and the like are laid in a poultry house, followed in general by being graded in accordance with the physical properties, such as weight, by an egg grading and packing apparatus and packaged with an egg box made of a clear synthetic resin or the like, and then distributed in the market. Prior to or in parallel with the grading and packing, eggs are subjected to various types of inspection.

As such egg inspection, there is so-called dirty egg inspection to detect eggs with a dirt for removal. An example of an apparatus to automate the inspection is a dirty egg inspection apparatus described in PTL 1. The dirty egg inspection apparatus determines a dirt on an eggshell surface, by radiating light from 300 nm to 500 nm to an egg and utilizing fluorescence in a visible light region (from 600 nm to 700 nm) to cause eggshell pigment on the eggshell surface to generate light at the above wavelengths as excited light, from an emphasized contrast between an image of an area with no dirt (fluorescing) and an image of an area with a dirt (not fluorescing).

### Citation List

### Patent Literature

[PTL 1] JP2005-127720 A

### Summary of Invention

### Technical Problem

To eggs, other than a non-transparent dirt, transparent egg white sometimes adheres. Although an egg with egg white is different from a dirty egg, there is a demand for removal from the perspective of hygiene because it contains protein and the like, being organic matters. Egg white is, however, transparent and the light from 300 nm to 500 nm passes therethrough. It is thus not possible to obtain a sufficient contrast for the eggshell surface and the dirty egg inspection apparatus described in PTL 1 is not capable of detecting egg white. That is, in examples, such as egg white, the dirty egg inspection measure described in PTL 1 sometimes does not function effectively as an egg inspection apparatus to inspect the state of adherence of protein to an eggshell surface and the like.

The present invention has been made to solve the problem and is to provide an egg inspection apparatus to perform egg inspection based on the presence of adherence of protein to an eggshell.

### Solution to Problem

According to the present invention, an egg inspection apparatus is provided that includes: a light radiating unit to radiate radiation light including an ultraviolet light component having a wavelength in an ultraviolet region to an eggshell surface of an egg subjected to inspection; a light receiving unit to receive reflection light reflecting from the eggshell surface out of the radiation light; and a determination unit to determine a state of adherence of protein to the eggshell surface based on the ultraviolet light component out of the reflection light received by the light receiving unit.

According to the egg inspection apparatus, radiation light including an ultraviolet light component having a wavelength in an ultraviolet region is radiated from the light radiating unit to the eggshell surface. The eggshell surface reflects many ultraviolet light components of the radiation light. In contrast, protein absorbs ultraviolet light components of the radiation light and reflects less ultraviolet light components. Therefore, when protein adheres to the eggshell surface, the ultraviolet light components that are included in the reflection light decrease. The determination unit determines a state of adherence of protein to the eggshell surface based on the ultraviolet light components out of the reflection light received by the light receiving unit. The egg inspection apparatus according to the present invention is thus capable of readily detecting protein adhering to an eggshell surface.

Various embodiments of the present invention are exemplified below. The embodiments below may be combined with each other.

It is preferred that the light receiving unit includes an ultraviolet photodetector to selectively detect a light component at a first specification wavelength out of the ultraviolet light component included in the reflection light, and the determination unit determines the state of the adherence of protein to the eggshell surface based on a value detected by the ultraviolet photodetector.

It is preferred that the first specification wavelength is a specific wavelength within a range from 200 nm to 300 nm.

It is preferred that the light receiving unit includes an ultraviolet light image capturing unit to generate an ultraviolet light image based on the ultraviolet light component included in the reflection light, and the determination unit determines the state of the adherence of protein to the eggshell surface based on an ultraviolet light strong adsorption region in the ultraviolet light image.

It is preferred that the radiation light includes a visible light component having a wavelength in a visible light region, and the determination unit determines a state of the egg based on the ultraviolet light component and the visible light component out of the reflection light received by the light receiving unit.

It is preferred that the light receiving unit includes a visible light detector to selectively detect a light component at a second specification wavelength out of the visible light component included in the reflection light, and the determination unit determines the state of the egg based on a value detected by the ultraviolet photodetector and a value detected by the visible light detector.

It is preferred that the second specification wavelength is a specific wavelength within a range from 400 nm to 500 nm.

It is preferred that the light radiating unit includes a sterilization lamp.

It is preferred that the light receiving unit includes a visible light image capturing unit to generate a visible light image based on the visible light component included in the reflection light, and the determination unit determines a state of a visible crack in the eggshell and adherence of visible dirt to the eggshell surface based on a visible light weak detection area in the visible light image.

It is preferred that the determination unit determines whether the visible light weak detection area is derived from the visible crack or the visible dirt based on contrast between inside and outside the visible light weak detection area.

It is preferred that the visible light weak detection area is specified by edge detection.

It is preferred that the determination unit determines whether or not the egg subjected to the inspection is a leaking egg based on the state of the adherence of protein to the eggshell surface, the state of the visible crack in the eggshell, and the state of the adherence of visible dirt to the eggshell surface.

It is preferred that the determination unit determines which of a normal egg, a dirty egg, a visibly cracked egg, and the leaking egg is the egg subjected to the inspection based on the state of the adherence of protein to the eggshell surface, the state of the visible crack in the eggshell, and the state of the adherence of visible dirt to the eggshell surface.

It is preferred that the apparatus further includes a transfer unit to transfer the egg subjected to the inspection while rotating, wherein the determination unit determines the state of the egg every time the egg is rotated by a predetermined degree.

According to the present invention, an egg inspection method is provided that uses the egg inspection apparatus described above, including the steps of: radiating radiation light including an ultraviolet light component having a wavelength in an ultraviolet region to an eggshell surface; receiving reflection light reflecting from the eggshell surface out of the radiation light; and determining a state of adherence of protein to the eggshell surface based on the ultraviolet light component out of the reflection light received by the light receiving unit.

### Brief Description of Drawings

Fig. 1 is a schematic view illustrating a scheme of an egg inspection apparatus according to a first embodiment of the present invention.
Figs. 2 are graphs illustrating peaks of light intensity relative to wavelengths from 200 nm to 500 nm in a sterilization lamp in Fig. 1, Fig. 2(A) being a graph where the scale of the ordinate is adapted to a peak around 254 nm and Fig. 2(B) being a graph where the scale of the ordinate is adapted to other peaks.
Fig. 3 is a schematic view illustrating a configuration of the light receiving unit in Fig. 1.
Figs. 4 are graphs illustrating results of inspection of a normal egg and an adhering egg by the egg inspection apparatus in Fig. 1.
Fig. 5 is a graph illustrating results of inspection of a normal egg, a wet egg, a dirty egg, an egg-white adhering egg, an egg-yolk adhering egg, and a leaking egg by the egg inspection apparatus in Fig. 1.
Fig. 6 is a graph illustrating optical absorption spectra of tryptophan (Trp), tyrosine (Try), phenylalanine (Phe), and albumin (BSA).
Fig. 7 is a schematic view illustrating a scheme of an egg inspection apparatus according to a second embodiment of the present invention.
Figs. 8 are illustrative diagrams of a method of analyzing an ultraviolet light image in the second embodiment of the present invention.
Fig. 9 illustrates results of a test for determination of the state of eggs subjected to inspection by the method in the second embodiment of the present invention.

### Description of Embodiments

### (First Embodiment)

Descriptions are given to an egg inspection apparatus 1 according to a first embodiment of the present invention with reference to the drawings. The egg inspection apparatus 1 is installed in, for example, a grading and packing system for eggs (e.g., refer to JP 2005-127720A) to be utilized for grading of an egg E. In this case, the egg inspection apparatus 1 detects an adhering substance adhering to an eggshell surface Ef of an egg transferred by the grading and packing system.

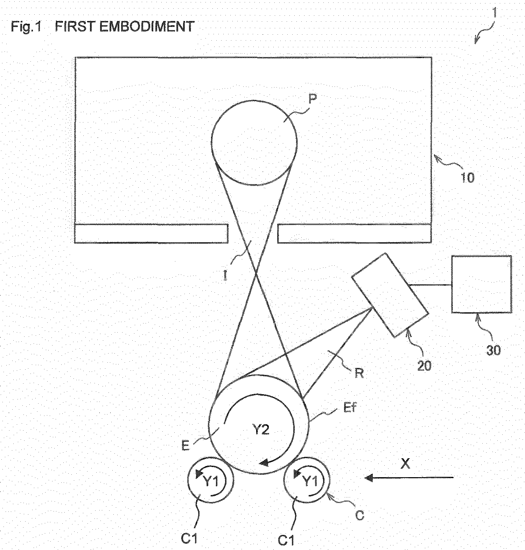

As illustrated in Fig. 1, the egg inspection apparatus 1 includes a light radiating unit 10 to radiate light to the eggshell surface Ef, a light receiving unit 20 to receive light from the eggshell surface Ef, and a determination unit 30 to determine presence of protein from the light received by the light receiving unit 20. The egg E subjected to inspection is inspected while being transferred by a transfer unit C of the grading and packing system. The transfer unit C is configured to hold the egg E between a pair of rollers C1 and rotate the egg E in an arrow Y2 direction with rotation of the rollers C1 in an arrow Y1 direction. As illustrated by an arrow X, the rollers C1 are capable of sliding movement. Therefore, the egg E moves in the arrow X direction while being rotated on the rollers C1. To inspect all around the egg E, the egg inspection apparatus 1 is configured to perform inspection every time the egg E is rotated by a predetermined degree (e.g., 60 degrees). The rollers C1 may be configured to rotate continuously or may be configured to stop every time rotating by a predetermined degree and rotate again after completion of egg inspection.

Radiation light I radiated from the light radiating unit 10 includes at least a light component from 200 nm to 300 nm, that is, a light component having a wavelength in part of the ultraviolet light region (from 190 nm to 400 nm). The radiation light I is radiated to the eggshell surface Ef. In addition, the light radiating unit 10 may include a light component having a wavelength in the visible light region from 400 nm to 800 nm. In particular, the light radiating unit 10 may radiate the radiation light I including a light component having a wavelength from 400 nm to 500 nm. In the present embodiment, the light radiating unit 10 is provided with a conventionally well-known sterilization lamp P as a light source. The light radiating unit 10 may be provided with another light source, such as an LED and a laser, as long as it is capable of radiating a light component in the above wavelength region.

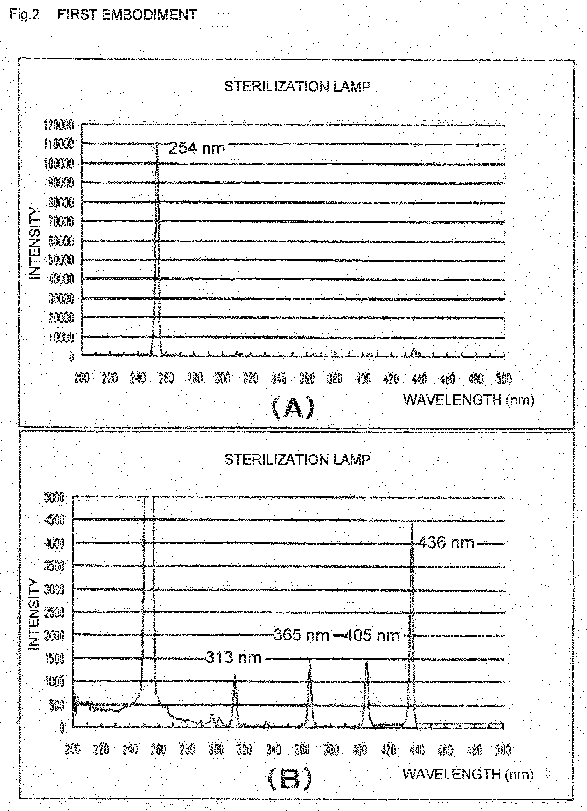

As illustrated in Figs. 2, the sterilization lamp P has peaks of the light intensity around 254 nm, around 313 nm, around 365 nm, around 405 nm, and around 436 nm, and the peak around 254 nm is a particularly large peak. The sterilization lamp P also has a relatively large peak at the peak around 436 nm. In the descriptions below, a light component around a specification wavelength is referred to as a "light component at a specification wavelength". That is, a "light component at 254 nm" means a light component around 254 nm.

Referring to Fig. 1 again, the light receiving unit 20 is arranged in a position to receive reflection light R from the eggshell surface Ef of the egg out of the radiation light I from the light radiating unit 10. The light receiving unit 20 is provided with at least one detector 22. In the present embodiment, the detector 22 is a conventionally well-known photodiode while it may be another device as long as it is capable of detecting light for conversion to an electrical signal. For example, the detector 22 may be a device, such as a CCD camera, that detects light for conversion to an electrical signal to obtain an image

Out of the radiation light I radiated to the eggshell surface Ef, many light components from 200 nm to 300 nm are reflected on the eggshell surface Ef of the egg. Accordingly, the reflection light R received by the light receiving unit 20 includes many components at wavelengths from 200 nm to 300 nm. The reflection light R is light that used to be the radiation light I reflected on the eggshell surface without converting the wavelength of the radiation light I, and the wavelengths of the radiation light I and the reflection light R are identical. In contrast, the fluorescence is light that is emitted from the eggshell by causing the radiation light I radiated to the eggshell surface to have a longer wavelength, and thus the reflection light R is different from fluorescence.

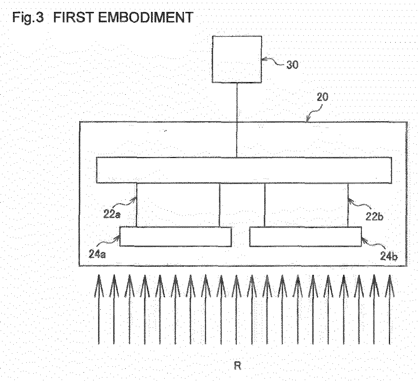

As illustrated in Fig. 3, in the present embodiment, the light receiving unit 20 is provided with an ultraviolet photodetector 22a and a visible light detector 22b. The ultraviolet photodetector 22a is provided with a bandpass filter 24a to pass a light component at 254 nm, and the visible light detector 22b is provided with a bandpass filter 24b to pass a light component at 436 nm. The ultraviolet photodetector 22a is thus capable of selectively detecting light components at 254 nm only, and the visible light detector 22b is capable of selectively detecting light components at 436 nm only.

In the present embodiment, by using bandpass filters that pass light components different from each other, the ultraviolet photodetector 22a and the visible light detector 22b are configured to allow reception of light components at wavelengths different from each other. However, the configuration to allow selective detection of a light component is not limited to this, and a light component at a specification wavelength may be selectively detected by configuring a detector using, for example, a device that is sensitive only in a specific wavelength region.

In the present embodiment, for example, the light radiating unit 10 radiates the radiation light I including a light component at 254 nm and the light receiving unit 20 selectively detects the light component at 254 nm in the ultraviolet photodetector 22a. By such configuration, the ultraviolet photodetector 22a only detects the reflection light R and does not detect, for example, fluorescence. That is, the light radiating unit 10 radiates the radiation light I including a light component at a specification wavelength, and the light receiving unit 20 selectively detects the light component at the specification wavelength in the detectors 22a and 22b. The detectors 22a and 22b therefore detect the reflection light R only. The light radiating unit 10 and the light receiving unit 20 are appropriately selected in such a manner that the detectors 22a and 22b only detect the reflection light R.

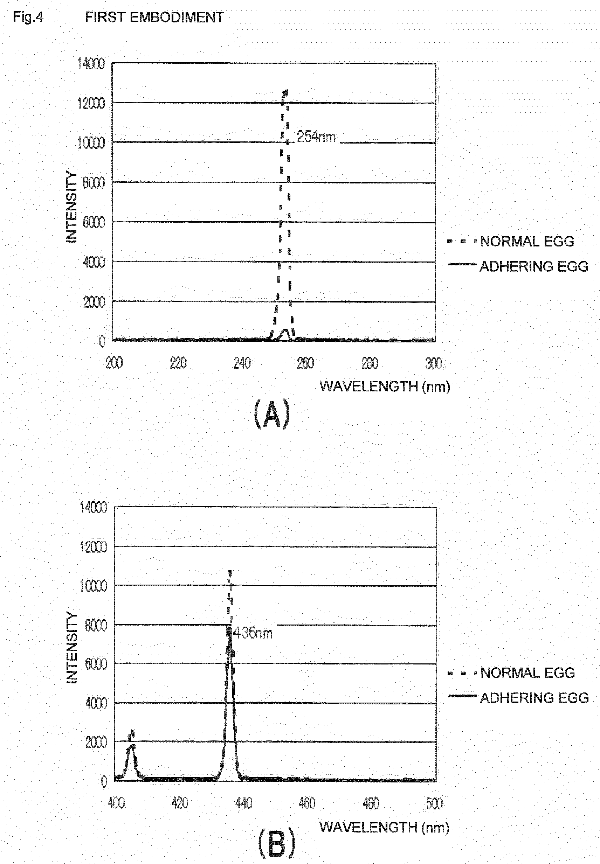

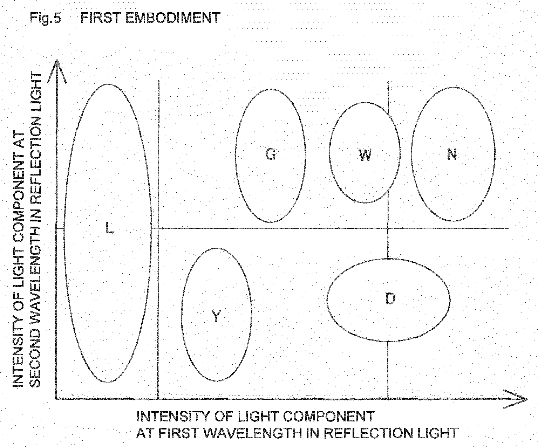

Information on the reflection light R detected by the light receiving unit 20 in the detectors 22a and 22b is converted to an electrical signal to be sent to the determination unit 30. The information to be sent to the determination unit 30 includes, for example, information on the intensity of the reflection light relative to wavelengths of light. From the information on the intensity of the reflection light relative to the wavelengths of light, graphs as illustrated in, for example, Figs. 4 and 5 are obtained.

Figs. 4 are graphs comparing an egg in a state where egg white adheres to the eggshell surface Ef (hereinafter, referred to as an "adhering egg") with an egg in a state where no adhering substance adheres (hereinafter, referred to as a "normal egg"). That is, in the egg inspection apparatus according to the present embodiment, Figs. 4 are graphs obtained by respectively inspecting an adhering egg with egg white adhering to the eggshell surface Ef and a normal egg without an adhering substance, such as egg white.

In Figs. 4, the ordinate indicates the intensity of light and the abscissa indicates the wavelengths of light. Fig. 4(A) illustrates the intensity of the light component from 200 to 300 nm detected by the detector, and Fig. 4(B) illustrates the intensity of the light component from 400 to 500 nm detected by the detector.

For both the adhering egg and the normal egg, in Fig. 4(A), a peak of the intensity of the reflection light is detected at 254 nm by the detector, and in Fig. 4(B), a peak of the intensity of the reflection light is detected at 436 nm by the detector. Because the light radiated from the sterilization lamp P has higher intensity at 254 nm and 436 nm and the reflection light received by the light receiving unit 20 thus has higher intensity at 254 nm and 436 nm, the peaks of the light intensity are at these wavelengths.

Although light components by the fluorescence from the eggshell are sometimes incident to the light receiving unit 20, the detectors 22a and 22b selectively receives light components at 254 nm and 436 nm and thus the light components by the fluorescence do not substantially affect information obtained from the reflection light R.

In Fig. 4(A), the peak of the normal egg at 254 nm is higher than the peak of the adhering egg at 254 nm. The reason is as follows. Out of the radiation light I from the light radiating unit 10, majority of the light component at 254 nm are reflected on the eggshell surface in the normal egg, whereas they are absorbed by the egg white in the adhering egg and are not reflected. In the normal egg, therefore, the reflection light R includes many light components at 254 nm and the intensity detected by the detector 22a is high, whereas in the adhering egg, the reflection light R includes less light components at 254 nm and the intensity detected by the detector 22a is low. Accordingly, Fig. 4(A) illustrates that, when an adhering egg is inspected, the intensity of the light component at 254 nm is less compared with the case of inspecting a normal egg.

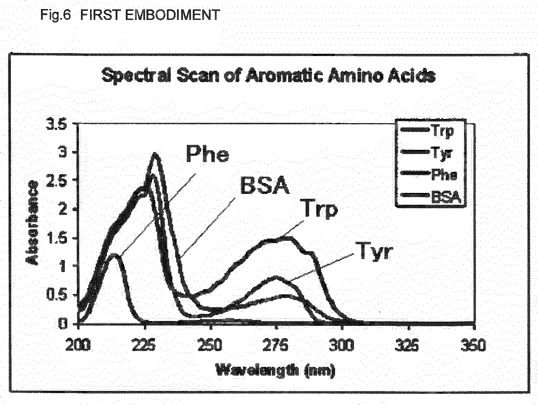

As conventionally well known, egg white and egg yolk include protein and protein has many types of amino acid. Such amino acid includes aromatic amino acid represented by tryptophan, and aromatic amino acid absorbs light in the ultraviolet light region. Fig. 6 illustrates optical absorption spectra of tryptophan (Trp), tyrosine (Try), phenylalanine (Phe), and albumin (BSA).

For example, as illustrated in Fig. 6, tryptophan absorbs light components from 200 to 300 nm and the reflection light R thus has less light components in the wavelength region. Accordingly, when light including a light component at a specific wavelength (e.g., 254 nm) in the ultraviolet light region is radiated to tryptophan, light at a wavelength longer than the specific wavelength (254 nm) is emitted as fluorescence while no light component at 254 nm is emitted. That is, since light components at 254 nm are absorbed by tryptophan, light components at 254 nm included in the reflection light R decreases.

Consequently referring to Figs. 4 again, even when light including a light component at 254 nm is radiated to an adhering egg for inspection of the adhering egg, egg white, that is, tryptophan absorbs the light components at 254 nm and the light components at 254 nm included in the reflection light R received by the light receiving unit 20 decreases, and thus the detector 22a barely detects the light components at 254 nm.

In contrast, for inspection of a normal egg, the eggshell surface Ef with no adhering substance has properties to reflect light components at 254 nm without absorbing as conventionally well known. When light including a light component at 254 nm is radiated to the normal egg, the eggshell surface thus reflects many light components at 254 nm and the reflection light R received by the light receiving unit 20 includes many light components at 254 nm, and the detector 22a detects many light components at 254 nm. Accordingly, in Fig. 4(A), the peak of the normal egg at 254 nm is higher than the peak of the adhering egg at 254 nm.

In Fig. 4(B), the peak of the normal egg at 436 nm is approximately equivalent to the peak of the adhering egg at 436 nm. As conventionally well known, egg white has transparent appearance and transmits light components in the visible light region. Accordingly, even in the case of the adhering egg with adhering egg white, egg white transmits light components at 436 nm in the visible light region and many of them are reflected on the eggshell surface Ef. Similarly, even in the case of the normal egg, many of the light components at 436 nm are reflected on the eggshell surface Ef.

Accordingly, in both cases of the normal egg and the adhering egg, the detector 22b detects many light components at 436 nm included in the reflection light R. The light components at 436 nm do not excite the egg, and fluorescence is not emitted from the eggshell surface Ef derived from the light components.

As just described, in Fig. 4(A), the adhering egg has a small peak at 254 nm and the normal egg has a large one, and in Fig. 4(B), there is no significant difference between the peaks at 436 nm of the adhering egg and the normal egg. Accordingly, the determination unit 30 of the egg inspection apparatus in the present embodiment is capable of determining a state of adherence of protein to the eggshell surface from the light components at 254 nm, which are light components in the ultraviolet light region, included in the reflection light R.

The determination unit 30 of the egg inspection apparatus in the present embodiment is capable of determining whether it is an adhering egg or a normal egg by combining information on intensity of light components having a wavelength in a visible region, for example at 436 nm, included in the reflection light R. That is, the type of the adhering substance on the eggshell surface is determined by combining a result of detection by the ultraviolet photodetector 22a and a result of detection by the visible light detector 22b.

A description is then given to Fig. 5. Fig. 5 illustrates a graph based on the information obtained by the determination unit 30, where the abscissa indicates intensity of the light component at 254 nm in the reflection light R and the ordinate indicates intensity of the light component at 436 nm in the reflection light R. The regions illustrated in Fig. 5 indicate distribution obtained when a plurality of eggs E with various adhering substances adhering to the eggshell surface Ef are inspected by the egg inspection apparatus 1 according to the present embodiment. That is, Fig. 5 is experimental results of inspecting adhering substances of eggs by the egg inspection apparatus.

Fig. 5 illustrates that, when an egg with a dirt, such as poultry manure, egg white, egg yolk, and water adhering thereto is inspected, it is possible to inspect the type of the adhering substance adhering to the eggshell from the result of detection by the detectors 22a and 22b. The egg may have, other than a dirt, egg white, egg yolk, and water adhering thereto without the eggshell being broken, the contents, that is, egg white or egg yolk discharged from the eggshell being broken to adhere to the eggshell surface Ef. This is referred to as a leaking egg and Fig. 5 illustrates that it is also possible to inspect the leaking egg.

Fig. 5 is a graph, where the abscissa indicates the intensity of the light component at 254 nm (first wavelength) in the reflection light R and the ordinate indicates the intensity of the light component at 436 nm (second wavelength) in the reflection light R, on which one point is plotted for each egg in accordance with the intensity of the light components at the first and second wavelengths in the reflection light from the egg with an adhering substance adhering thereto and the leaking egg. Accordingly, one plot is obtained for one egg. Since the position of the plot for each egg correlates with the type of the adhering substance and the presence of leaking contents, a plurality of plots are unevenly distributed in a specific region for each state of egg. In Fig. 5, the plots of the respective eggs are omitted and the regions in which the plots are unevenly distributed are illustrated.

Fig. 5 illustrates, as the eggs to be inspected by the egg inspection apparatus 1, a normal egg N with no adhering substance including leakage of the contents, a dirty egg D with an adhering dirt, a wet egg W with adhering water, an egg-white adhering egg G with adhering egg white, an egg-yolk adhering egg Y with adhering egg yolk, and a leaking egg L with adhering contents (egg white or egg yolk).

The normal egg N absorbs less light components at the first and second wavelengths and largely reflects them on the eggshell surface Ef, it is plotted in the upper right where the intensity of the light components at both wavelengths is large. Accordingly in Fig. 5, when the normal egg N is inspected, the plot is unevenly distributed in an upper right region N.

The dirty egg D with an adhering dirt generally has colored substance adhering as the adhering substance. Examples of the adhering substance include poultry manure, soil, and the like. Since poultry manure, soil, and the like do not contain protein, majority of the light components at the first wavelength are not absorbed by the adhering substance. Meanwhile, since the adhering substance is a colored substance, majority of the light components at the second wavelength are absorbed by the adhering substance. Accordingly, in the reflection light R, the intensity of the light components at the first wavelength is large and the intensity of the light components at the second wavelength is small. Accordingly in Fig. 5, when the dirty egg D is inspected, a result of inspecting the dirty egg D is plotted in uneven distribution in a lower right region D.

The water adhering to the wet egg W does not contain protein and is transparent. The light components of both the first and second wavelengths are accordingly reflected on the eggshell surface Ef without being largely absorbed by the water. The intensity of the light components at the first and second wavelengths in the reflection light R thus increases. In Fig. 5, a result of inspecting the wet egg W is accordingly plotted in uneven distribution in an upper right region W. It should be noted that it is plotted in a region slightly on the left compared with that of the normal egg N due to the partial scattering and the like of the light by the water.

The egg white adhering to the egg-white adhering egg G contains protein and is transparent. Consequently, the light components at the first wavelength are largely absorbed by the egg white. The light components at the second wavelength are reflected on the eggshell surface Ef without being largely absorbed by the egg white. Thus in the reflection light R, the intensity of the light components at the first wavelength decreases, and the intensity of the light components at the second wavelength increases. Accordingly in Fig. 5, a result of inspecting the egg-white adhering egg G is plotted in uneven distribution in a upper central region G.

The egg yolk adhering to the egg-yolk adhering egg Y contains protein and is colored. The light components of both the first and second wavelengths are thus absorbed by the egg yolk. The intensity of the light components at the first and second wavelengths in the reflection light R thus decreases. Accordingly in Fig. 5, a result of inspecting the egg-yolk adhering egg Y is plotted in uneven distribution in a lower central region Y.

In the leaking egg L discharging the contents, egg white or egg yolk adheres to the eggshell surface Ef in a larger amount compared with that of the egg-white adhering egg or the egg-yolk adhering egg. Accordingly, the light components at the first wavelength are largely absorbed by the large amount of egg white or egg yolk and are not reflected. When the leaking contents are egg white, the light components at the second wavelength are reflected on the eggshell surface Ef without being largely absorbed by the egg white. In contrast, when the leaking contents are egg yolk, the components are absorbed by the egg yolk.

Accordingly, in the leaking egg L, the intensity of the light components at the first wavelength in the reflection light R decreases very much and the intensity of the light components at the second wavelength changes in accordance with the leaking contents. In Fig. 5, a result of inspecting the leaking egg L is plotted in uneven distribution on the left because the light components at the first wavelength are absorbed and plotted in uneven distribution in a vertically extended region L in accordance with absorption of the light components at the second wavelength generated by the leaking contents. More egg yolk leakage causes uneven distribution of the plots in a lower part in the region L.

With the adhering substance adhering to the eggshell surface Ef or the leaking contents of the leaking egg L, the region plotted in Fig. 5 is specified. This enables determination of the type of the adhering substance or whether or not the egg is a leaking egg. It is accordingly possible to utilize the egg inspection apparatus according to this embodiment of the present invention as a so-called leaking egg inspection apparatus.

In the experiment of Fig. 5, a leaking egg is considered separately from an egg-white adhering egg or an egg-yolk adhering egg while, even to a leaking egg, egg yolk or egg white adheres. It is accordingly possible to determine a leaking egg as an adhering egg. A graph of adhering substances as that in Fig. 5 is an exemplification, and the regions to classify the plots of eggs with various adhering substances adhering thereto are selected appropriately. That is, the determination unit may determine the type of the adhering substance by another graph, another mechanism, and the like as long as it is capable of inspecting the adhering substance on the eggshell surface from the intensity of the light components at the first and second wavelengths.

The determination unit 30 is configured with conventionally well-known software and hardware. The positional relationship of the light radiating unit 10, the light receiving unit 20, the transfer unit C, and the egg E may be appropriately modified to be suitable for detection of the adhering substance. To facilitate detection of the adhering substance, a configuration, such as covering the entire device with a shading box, may be added appropriately. Further, the light intensity in Figs. 2, 4, and 5 is modified by the positional relationship of the radiating unit 10, the light receiving unit 20, and the egg E and the like and thus it is appropriately adjusted in accordance with the inspection.

The wavelength of the light component to be selectively detected by the light receiving unit 20 may be modified appropriately. For example, when the adhering substance is capable of absorbing light at 250 nm, the light receiving unit 20 to receive the reflection light R may selectively detect a light component at 250 nm using light including a light component at 250 nm as the radiation light I.

Using an ultraviolet camera (ultraviolet light image capturing unit) that is sensitive to an ultraviolet light component as the detector 22a, an optical filter at 254 nm or the like may be provided in front of the ultraviolet camera. This enables the camera to obtain an ultraviolet light image by receiving an ultraviolet light component only and to determine the state of adherence of protein from the image.

In the present embodiment, the light radiating unit 10 is composed of the sterilization lamp P having peaks of the light intensity at a plurality of wavelengths while it may include at least a light component from 200 nm to 300 nm even without peaks at a plurality of wavelengths.

In the present embodiment, when determining an adhering substance represented by protein, the determination unit 30 may determine with reference to a value investigated in advance or may determine by comparing a value obtained from the egg subjected to inspection with a value obtained from another egg out of the plurality of eggs to be inspected.

In the present embodiment, the determination unit 30 determines an adhering substance by comparing the intensity of the light component at a specification wavelength between the radiation light I and the reflection light R while it may determine an adhering substance by another conventionally well-known method, such as to determine an adhering substance by comparing the spectra between the radiation light I and the reflection light R, for example.

### (Second Embodiment)

In the first embodiment, whether an egg E subjected to inspection is a leaking egg or an adhering egg is determined using the graph illustrated in Fig. 5. Note that, in the first embodiment, the determination is made based on the intensity of the light components at the first and second wavelengths in the reflection light R. When the leakage of egg white or egg yolk is relatively little even the egg is a leaking egg, for example, absorption of the light components at 254 nm on the eggshell surface is thus relatively little, and as a result, there is a problem that a leaking egg is sometimes determined by mistake as an egg-white or egg-yolk adhering egg. The present embodiment has been made to solve such problem and determines whether the egg subjected to the inspection is a leaking egg with higher accuracy. A detailed description follows.

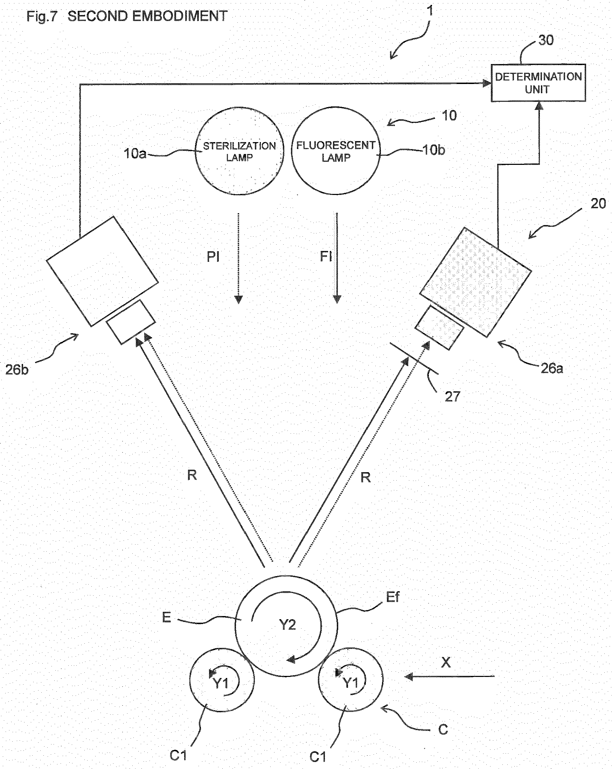

In the present embodiment, as illustrated in Fig. 7, an egg inspection apparatus 1 includes the light radiating unit 10 to radiate light to the eggshell surface Ef, a light receiving unit 20 to receive light from the eggshell surface Ef, and a determination unit 30 to determine presence of protein from the light received by the light receiving unit 20. The egg E subjected to inspection is inspected while being transferred by a transfer unit C of a grading and packing system. The configuration of the transfer unit C is same as that in the first embodiment.

The light radiating unit 10 includes a first light radiating unit 10a to radiate first radiation light PI to the egg E and a second light radiating unit 10b to radiate second radiation light FI to the egg E. The first radiation light PI is light having an ultraviolet light component as a main component, and the second radiation light FI is light having a visible light component as a main component. Accordingly, the intensity of the ultraviolet light component in the first radiation light PI is higher than that of the ultraviolet light component in the second radiation light FI, and the intensity of the visible light component in the second radiation light FI is higher than that of the ultraviolet light component in the first radiation light PI. In the same manner as the first embodiment, the first light radiating unit 10a is configured with a sterilization lamp and the like, and the second light radiating unit 10b is configured with, for example, a fluorescent lamp. Here, two types of radiation light with different wavelength distribution are emitted from the two light radiating units and both types of radiation light are radiated to the egg E, thereby enabling radiation of the radiation light including both the ultraviolet light component and the visible light component to the egg E. In another embodiment, a single light radiating unit to emit radiation light including both the ultraviolet light component and the visible light component may be used.

The reflection light R generated by reflecting the radiation light PI and FI on the eggshell surface Ef of the egg E includes, in the same manner as the radiation light PI and FI, an ultraviolet light component and a visible light component. The light receiving unit 20 is provided with an ultraviolet light image capturing unit 26a and a visible light image capturing unit 26b. Between the ultraviolet light image capturing unit 26a and the egg E, a visible light blocking filter 27 to cut visible light components is arranged. Only ultraviolet light components in the reflection light R therefore reach the ultraviolet light image capturing unit 26a, and the ultraviolet light image capturing unit 26a generates an ultraviolet light image based on the ultraviolet light components included in the reflection light R. In the present embodiment, the visible light blocking filter 27 is configured to transmit only light components at a specification wavelength (e.g., 254 nm), whereas it may be configured to transmit light components in a specific wavelength range (e.g., from 200 to 300 nm). As the visible light blocking filter 27, those same as the bandpass filter 24a in the first embodiment may be used.

In contrast, between the visible light image capturing unit 26b and the egg E, no filter is arranged and all components of the reflection light R reach the visible light image capturing unit 26b. However, by using those less sensitive to the ultraviolet light components than to the visible light components as the visible light image capturing unit 26b (preferably, substantially not sensitive to the ultraviolet light components), the visible light image capturing unit 26b is capable of generating a visible light image based on the visible light components included in the reflection light R. In addition, between the visible light image capturing unit 26b and the egg E, an ultraviolet light blocking filter to cut ultraviolet light components may be arranged. In this case, those sensitive to ultraviolet light components may be utilized for the visible light image capturing unit 26b. In addition, the ultraviolet light blocking filter may be configured to transmit only light components at a specification wavelength in the visible light region or to transmit light components in a specific wavelength range (e.g., from 400 to 500 nm). As the ultraviolet light blocking filter, those same as the bandpass filter 24b in the first embodiment may be used.

The ultraviolet light image and the visible light image are sent to the determination unit 30 for image analysis and the state of the egg E is determined based on the results.

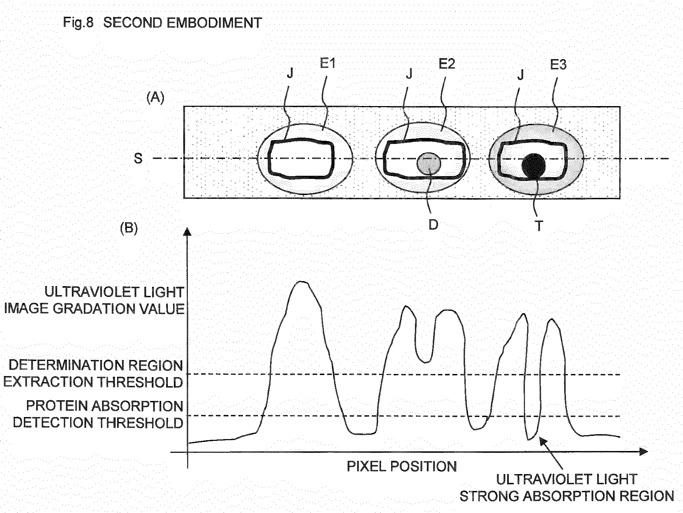

Here, with reference to Figs. 8, image analysis of ultraviolet light images is described. Fig. 8(A) illustrates ultraviolet light images captured of three eggs E1 through E3. The egg E1 has no adhering substance, the egg E2 has an adhering dirt D, such as cecal feces, not containing a lot of protein, and the egg E3 has adhering protein T, such as egg white. Fig. 8(B) is a graph illustrating gradation values (brightness values) of the ultraviolet light images in pixel positions on a straight line S in Fig. 8(A). On the ordinate in Fig. 8(B), a determination region extraction threshold and a protein absorption detection threshold are set. A region where the gradation values of the ultraviolet light images exceed the determination region extraction threshold is set as determination regions J illustrated in Fig. 8(A). In the determination regions J, a region where the gradation values of the ultraviolet light images fall below the protein absorption detection threshold is set as an ultraviolet light strong absorption region. In a region where protein adheres, since the gradation values of the ultraviolet light component are greatly reduced due to the absorption of the ultraviolet light component by the protein, the ultraviolet light strong absorption region corresponds to the region where the protein adheres.

In the egg E1 with no adhering substance, since no ultraviolet light strong absorption region is present in the determination regions J, determination is made that the egg E1 has no adhering protein. In the egg E2 with the adhering dirt D, although there is a depression in the gradation values in the determination regions J, the lower limit of the gradation values in the depression is larger than the protein absorption detection threshold and thus determination is made that the egg E2 has no adhering protein. In contrast, in the egg E3 where the protein T adheres, there is a depression in the gradation values in the determination regions J and the lower limit of the gradation values in the depression falls below the protein absorption detection threshold. Determination is thus made that there is the ultraviolet light strong absorption region in the ultraviolet light image and the egg E3 has adhering protein. In such a manner, in the present embodiment, the determination unit 30 determines the state of adherence of protein to the eggshell surface Ef based on the ultraviolet light strong absorption region in the ultraviolet light image. The determination unit 30 may determine that protein adheres when there is any ultraviolet light strong absorption region and that protein adheres when the ratio of the area of the ultraviolet light strong absorption region exceeds a threshold.

Then, image analysis of visible light images is described. A visible dirt and a visible crack in the visible light image, compared with an eggshell, absorb many visible light components or reflect or scatter the visible light components in directions other than the visible light image capturing unit 26b. The region where a visible dirt or a visible crack is present thus becomes a visible light weak detection area where visible light components are detected relatively weakly by the visible light image capturing unit 26b. The visible light weak detection area is specifiable by edge detection. In general, in the visible light weak detection area derived from a visible dirt, contrast between inside and outside the visible light weak detection area tends to be greater than that in the visible light weak detection area derived from a visible crack. Accordingly, whether or not the contrast exceeds a threshold may determine whether the visible light weak detection area is derived from a visible dirt or a visible crack. The determination whether the visible light weak detection area is derived from a visible dirt or a visible crack may be performed by considering the area or the color of the visible light weak detection area. The term "a visible crack" herein includes not only a crack in an eggshell but also a hole formed by a partial loss of an eggshell. In addition, "a visible crack" and "a visible dirt" herein mean, respectively, a crack and a dirt that are detectable by image analysis of a visible light image.

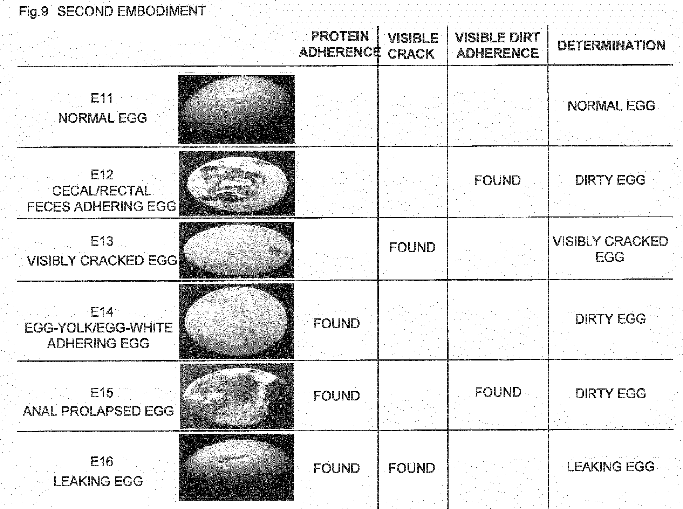

As just described, by analyzing ultraviolet light images and visible light images, it is possible to obtain (1) the state of adherence of protein to the eggshell surface, (2) the state of a visible crack in the eggshell, and (3) the state of adherence of visible dirt to the eggshell surface of the egg subjected to inspection. Then, based on these states, it is possible to determine the state of the egg subjected to inspection. Fig. 9 illustrates an example of determination of the state of an egg. In Fig. 9, determination is made for eggs E11 through E16.

The egg E11 is a normal egg without adherence of a dirt of protein and others and has no visible crack. When the inspection in the present embodiment was performed on the egg E11, adherence of protein was not detected by the ultraviolet light image analysis and neither a visible crack nor adherence of visible dirt was detected by the visible light image analysis. The determination unit 30 therefore determined that the egg E11 was "a normal egg".

The egg E12 is a cecal/rectal feces adhering egg that has adhering cecal/rectal feces but has no visible crack. When the inspection in the present embodiment was performed on the egg E12, adherence of protein was not detected by the ultraviolet light image analysis and adherence of visible dirt was detected by the visible light image analysis. The determination unit 30 therefore determined that the egg E12 was "a dirty egg".

The egg E13 is a visibly cracked egg and has no adherence of a dirt of protein and others but has a visible crack. When the inspection in the present embodiment was performed on the egg E13, adherence of protein was not detected by the ultraviolet light image analysis and a visible crack was detected by the visible light image analysis. The determination unit 30 therefore determined that the egg E13 was "a visibly cracked egg".

The egg E14 is an egg-yolk/egg-white adhering egg and has adhering protein but has no visible crack. When the inspection in the present embodiment was performed on the egg E14, adherence of protein was detected by the ultraviolet light image analysis and neither a visible crack nor adherence of visible dirt was detected by the visible light image analysis. The determination unit 30 therefore determined that the egg E14 was "a dirty egg". In the egg E14, the region where egg yolk adheres is yellowish and is more or less visually recognizable. In the visible light image, however, the contrast between the egg-yolk adherence region and the surrounding region falls below a predetermined threshold and thus the egg-yolk adherence region was not detected as the visible light weak detection area. As a result, neither a visible crack nor adherence of visible dirt was detected.

The egg E15 is an anal prolapsed egg and has protein and a visible dirt that adhere thereto but has no visible crack. When the inspection in the present embodiment was performed on the egg E15, adherence of protein was detected by the ultraviolet light image analysis, and adherence of visible dirt was detected and no visible crack was detected by the visible light image analysis. The determination unit 30 therefore determined that the egg E15 was "a dirty egg".

The egg E16 is a leaking egg with adhering protein and a visible crack. When the inspection in the present embodiment was performed on the egg E16, adherence of protein was detected by the ultraviolet light image analysis, and adherence of visible dirt was not detected and a visible crack was detected by the visible light image analysis. The determination unit 30 therefore determined that the egg E16 was "a leaking egg".

As just described, by the method in the present embodiment, it was possible to accurately determine the states of the six eggs E11 through E16 that were in states different from each other. Although determination was made here that the egg subjected to inspection was which of a normal egg, a dirty egg, a visibly cracked egg, and the leaking egg, the determination may be made, more simply, only whether the egg subjected to the inspection is a leaking egg.

In the present embodiment, the state of the egg subjected to inspection is determined based on (1) the state of adherence of protein to the eggshell surface, (2) the state of a visible crack in the eggshell, and (3) the state of adherence of visible dirt to the eggshell surface. Since it is therefore possible to detect even a leaking egg having relatively less leakage of protein, according to the present embodiment, it is possible to determine the state of the egg with higher accuracy than in the first embodiment.

In addition, although the state of adherence of protein is determined by the ultraviolet light image analysis in the present embodiment, in the same manner as the first embodiment, the state of adherence of protein may be determined based on a value detected by the ultraviolet photodetector 22a.

The embodiments disclosed herein are for exemplification and embodiments are not limited to them. The present invention is defined by the claims not by the scope of above description and is intended to include all modifications within the meaning and the scope of equivalents of the claims.

In the first and second embodiments, the criteria to determine whether an egg subjected to inspection falls under a protein adhering egg, a dirty egg, a leaking egg, a visibly cracked egg, or the like are for exemplification and the determination may be made in accordance with other determination criteria based on a combination of the intensity of each detected light component, adherence of protein to the eggshell surface, the state of a visible crack and a visible dirt, and the like.

### Industrial Applicability

The present invention is applicable to egg inspection.

### Reference Signs List

1: Egg Inspection Apparatus,
10: Light Radiating Unit,
20: Light Receiving Unit,
22: Detector,
24: Bandpass Filter,
30: Determination Unit.

## Claims

1. An egg inspection apparatus, comprising:
a light radiating unit to radiate radiation light including an ultraviolet light component having a wavelength in an ultraviolet region to an eggshell surface of an egg subjected to inspection;
a light receiving unit to receive reflection light reflecting from the eggshell surface out of the radiation light; and
a determination unit to determine a state of adherence of protein to the eggshell surface based on the ultraviolet light component out of the reflection light received by the light receiving unit.

2. The egg inspection apparatus according to claim 1, wherein
the light receiving unit includes an ultraviolet photodetector to selectively detect a light component at a first specification wavelength out of the ultraviolet light component included in the reflection light, and
the determination unit determines the state of the adherence of protein to the eggshell surface based on a value detected by the ultraviolet photodetector.

3. The egg inspection apparatus according to claim 2, wherein the first specification wavelength is a specific wavelength within a range from 200 nm to 300 nm.

4. The egg inspection apparatus according to any one of claims 1 through 3, wherein
the light receiving unit includes an ultraviolet light image capturing unit to generate an ultraviolet light image based on the ultraviolet light component included in the reflection light, and
the determination unit determines the state of the adherence of protein to the eggshell surface based on an ultraviolet light strong absorption region in the ultraviolet light image.

5. The egg inspection apparatus according to any one of claims 1 through 4, wherein
the radiation light includes a visible light component having a wavelength in a visible light region, and
the determination unit determines a state of the egg based on the ultraviolet light component and the visible light component out of the reflection light received by the light receiving unit.

6. The egg inspection apparatus according to claim 5, wherein
the light receiving unit includes a visible light detector to selectively detect a light component at a second specification wavelength out of the visible light component included in the reflection light, and
the determination unit determines the state of the egg based on a value detected by the ultraviolet photodetector and a value detected by the visible light detector.

7. The egg inspection apparatus according to claim 6, wherein the second specification wavelength is a specific wavelength within a range from 400 nm to 500 nm.

8. The egg inspection apparatus according to any one of claims 1 through 7, wherein the light radiating unit includes a sterilization lamp.

9. The egg inspection apparatus according to any one of claims 1 through 8, wherein
the light receiving unit includes a visible light image capturing unit to generate a visible light image based on the visible light component included in the reflection light, and
the determination unit determines a state of a visible crack in the eggshell and adherence of visible dirt to the eggshell surface based on a visible light weak detection area in the visible light image.

10. The egg inspection apparatus according to claim 9, wherein the determination unit determines whether the visible light weak detection area is derived from the visible crack or the visible dirt based on contrast between inside and outside the visible light weak detection area.

11. The egg inspection apparatus according to claim 9 or 10, wherein the visible light weak detection area is specified by edge detection.

12. The egg inspection apparatus according to any one of claims 9 through 11, wherein the determination unit determines whether or not the egg subjected to the inspection is a leaking egg based on the state of the adherence of protein to the eggshell surface, the state of the visible crack in the eggshell, and the state of the adherence of visible dirt to the eggshell surface.

13. The egg inspection apparatus according to claim 12, wherein the determination unit determines which of a normal egg, a dirty egg, a visibly cracked egg, and the leaking egg is the egg subjected to the inspection based on the state of the adherence of protein to the eggshell surface, the state of the visible crack in the eggshell, and the state of the adherence of visible dirt to the eggshell surface.

14. The egg inspection apparatus according to any one of claims 1 through 13, further comprising a transfer unit to transfer the egg subjected to the inspection while rotating, wherein
the determination unit determines the state of the egg every time the egg is rotated by a predetermined degree.

15. An egg inspection method using the egg inspection apparatus according to any one of claims 1 through 14, comprising the steps of:
radiating radiation light including an ultraviolet light component having a wavelength in an ultraviolet region to an eggshell surface;
receiving reflection light reflecting from the eggshell surface out of the radiation light; and
determining a state of adherence of protein to the eggshell surface based on the ultraviolet light component out of the reflection light received by the light receiving unit.
